# EUROPÄISCHE PATENTSCHRIFT

(11) **EP 1 549 955 B1**
(45) Veröffentlichungstag und Bekanntmachung des Hinweises auf die Patenterteilung: **25.04.2007**
(21) Anmeldenummer: 03757688.1
(22) Anmeldetag: 30.09.2003
(51) Int. Cl.: G01N 33/543

(54) **VERFAHREN ZUM NACHWEIS VON ANALYTEN**
METHOD FOR DETECTING ANALYTES
PROCEDE PERMETTANT DE DECELER DES ANALYTES

(30) Priorität: 30.09.2002 DE 10245644
(43) Veröffentlichungstag der Anmeldung: 06.07.2005
(73) Patentinhaber: Cibitest GmbH & Co. KG, 89231 Neu-Ulm (DE)
(72) Erfinder: BONENBERGER, Johannes, 87437 Kempten (DE); HAGENMAIER, Sigrid, 89081 Ulm (DE); VON KAMPEN, Jan, verstorben (DE); POLACKOVA, Jitka, 89231 Neu-Ulm (DE)
(74) Vertreter: Dehmel, Albrecht
(86) Internationale Anmeldenummer: PCT/DE2003/003253
(87) Internationale Veröffentlichungsnummer: WO 2004/031771

(56) Entgegenhaltungen:
- EP-A- 0 811 842
- WO-A-95/15981
- US-A- 5 516 635
- US-A1- 2002 001 853

## Beschreibung

Die vorliegende Erfindung betrifft ein Verfahren zum Nachweis von Analyten, umfassend die Schritte: Inkubieren einer Probe mit Makromolekülen, an die jeweils mindestens 2 Moleküle des in der Probe nachzuweisenden Analyten gekoppelt sind, anschließendes Inkubieren der Probe mit einem festen Träger, an den Einfangmoleküle gegen den nachzuweisenden Analyten gekoppelt sind, Zugabe eines die Makromoleküle anfärbenden Fluoreszenzfarbstoffes und Nachweis von in der Probe vorhandenen Analyten durch Anregung des Fluoreszenzfarbstoffes.

Für den Nachweis von niedermolekularen Analyten sind zahlreiche Verfahren etabliert. Diese finden in speziellen Laboratorien z.B. in der Suche nach Wirkstoffen für die pharmazeutische Industrie beim sog. High-Throughput-Screening (HTS) oder Very-High-Throughput-Screening (VHTS), aber auch in Vor-Ort-Testsystemen z.B. bei Drogen-, Umwelt- oder Lebensmittelschnelltests Anwendung. Dabei werden einerseits einige hundert (HTS) bis mehrere tausend (VHTS) "capture molecules" genannte Ziel- oder Einfangmoleküle, meist DNA oder Proteine, auf Objektträgern in sog. Microarrays immobilisiert, wobei derzeit je nach Art der immobilisierten Moleküle hauptsächlich zwischen DNA-Arrays und Proteinarrays unterschieden wird. Die Wechselwirkung der immobilisierten Biomoleküle kann mit den vorher markierten Inhaltsstoffen einer Untersuchungsmatrix, z.B. dem Lysat eines bestimmten Gewebetyps, untersucht werden.

Die Nachweisverfahren beim HTS oder VHTS basieren oftmals auf Fluoreszenzmarkierungen. Diese sind in der chemischen, biochemischen und biologischen Analytik seit längerem bekannt, z.B. in der Verwendung von FIA's (Fluorescent Immuno Assay), die in Mikrotiterplatten bzw. auf Biochips im Objektträgerformat durchgeführt werden. Die hierfür verwendeten Fluoreszenzfarbstoffe sind in der Regel sehr kurzlebig und benötigen eine intensive Anregung mit einer definierten Wellenlänge und emittieren in der Regel in einer nahegelegenen Wellenlänge, so dass ein optischer Filter zwischen Anregung und Emission geschaltet werden muss und das emittierte Licht von einer optischen Auswerteeinheit (Fluoreszenzreader etc.) erfasst werden muss. Aufgrund der minimalen Mengen an eingesetzten Analyten und deren räumlichen Dichte ist die Auswertung der Microarrays an hochwertige optische Geräte, z.B. Fluoreszenzmikroskope, und computergestützte Bildauswertungsprogramme gebunden.

Bedingt durch die aufwendige und empfindliche technologische Detektion werden die gängigen Fluoreszenzmarkierungen für Vor-Ort-Testsysteme, z.B. Drogen- und Umweltschnelltests, zur Zeit nicht verwendet. Diese vor Ort durchführ- und auswertbaren Testsysteme zeichnen sich durch ihre Robustheit gegenüber Anwendung durch ungeschultes Personal, widrigen Umwelteinflüssen und eine direkte, visuelle Auswertbarkeit mit dem menschlichen Auge aus. Die für diese Testsysteme gängigen Markierungsverfahren sind Kopplungen von Biomolekülen an Goldpartikel, farbige Polystrol- oder Latex-Beads, Liposomen oder Enzyme. All diese Markierungsverfahren benötigen ein weißes oder möglichst helles Trägermaterial, auf dem die Reaktionspartner immobilisiert werden, um nach der Reaktion mit einem markierten Reaktanten einen möglichst hohen Kontrast zu erzeugen. Daher sind diese in der Schnellanalytik gängigen Markierungen für Arrays auf klaren bzw. durchsichtigen Trägermaterialien nicht realisierbar. Ein weiterer Nachteil dieser visuell auswertbaren Markierungsverfahren ist die schlechte Auswertbarkeit im Dunkeln bzw. bei Kunstlicht, die aber für viele Anwendungen, wie z.B. bei der nächtlichen Drogenkontrolle von Autofahrern oder der Auswertung von Schadstofftests im Lebensmittelbereich, z.B. unter Stallbedingungen, unbedingte Voraussetzung ist.

Daher liegt der Erfindung die Aufgabe zu Grunde, ein sensitives, schnelles, vor Ort und unter ungünstigen Umgebungsbedingungen einfach durchzuführendes Verfahren zum Nachweis von chemischen Substanzen bereitzustellen.

Die Aufgabe wird durch den in den Patentansprüchen definierten Gegenstand gelöst.

Die nachfolgenden Figuren erläutern die Erfindung.

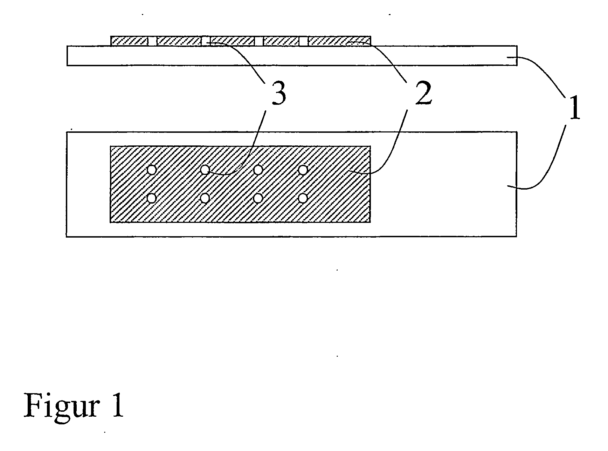

Fig. 1 zeigt einen Testträger im Querschnitt und in Aufsicht. Der eigentliche Testträger (1) ist mit einer Beschichtung (2) versehen, in die die Einfangmoleküle (3) eingebunden werden.

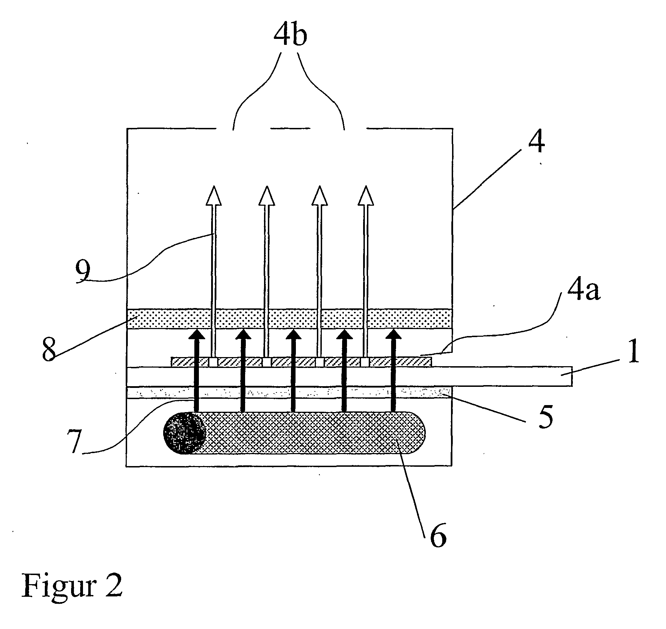

Fig. 2 zeigt eine Apparatur mit der die Testträger nach dem Testablauf ausgewertet werden können. Der Testträger (1) wird über eine Zuführung (4a) in das Gehäuse (4) der Apparatur geschoben. Unter dem Testträger in der Apparatur befinden sich eine Lichtquelle (6) und eine Filterplatte (5), die nur Licht im Bereich des Anregungsmaximums (7) des für den Nachweis herangezogenen Fluoreszenzfarbstoffs durchläßt. Über dem Testträger befindet sich eine zweite Filterplatte (8), die das Licht zur Anregung (7) abfängt und das vom Fluoreszenzfarbstoff emittierte Licht (9) durchläßt. Die Stellen an denen eine Fluoreszenzemission auf dem Testträger stattfindet, kann je nach verwendetem Farbstoff visuell oder über geeignete optische Instrumente durch Öffnungen (4b) im Gehäuse beobachtet werden.

Der Begriff "Analyt" wie hier verwendet bezeichnet die im vorliegenden Verfahren zu untersuchende Substanz.

Der Begriff "capture molecule" oder "Zielmolekül" oder "Einfangmolekül" wie hier verwendet bezeichnet das im vorliegenden Verfahren auf die Beschichtung des festen Trägers aufgetragene Molekül, an das ein freier oder gekoppelter Analyt binden kann.

Zur Lösung der Aufgabe werden nachfolgend Verfahren und Vorrichtungen beschrieben, die es erlauben Analyten, vorzugsweise niedermolekulare Analyten, unter Verwendung von Fluoreszenzfarbstoffen nachzuweisen.

Die vorliegende Erfindung betrifft ein Verfahren zum Nachweis von Analyten, umfassend die Schritte:
a) Inkubieren einer Probe mit Makromolekülen, an die jeweils mindestens 2 Moleküle des in der Probe nachzuweisenden Analyten gekoppelt sind,
b) anschließendes Inkubieren der Probe mit einem festen Träger, an den Einfangmoleküle gegen den nachzuweisenden Analyten gekoppelt sind,
c) Zugabe eines die Makromoleküle anfärbenden Fluoreszenzfarbstoffes und
d) Nachweis von in der Probe vorhandenen Analyten durch Anregung des Fluoreszenzfarbstoffes.

In dem erfindungsgemäßen Verfahren konkurrieren somit die in der Probe vorhandenen nachzuweisenden Analyten mit den an die Makromoleküle gebundenen Moleküle, die mit den Analyten identisch sind, um die Bindung an die Einfangmoleküle. Sind in der Probe die nachzuweisenden Analyten vorhanden, so werden die an die Makromoleküle gebundenen Analyten von der Bindung an die Einfangmoleküle abgehalten. Eine Anregung der Fluoreszenzfarbstoffe führt daher nicht zu einem positiven Signal. Sind dagegen in der Probe keine nachzuweisenden Analyten vorhanden, binden die an die Makromoleküle gebundenen Analyten an die Einfangmoleküle und die Anregung der Fluoreszenzfarbstoffe führt zu einem positiven Signal.

Gegebenenfalls kann nach Schritt c) der Schritt c') eingefügt werden: Entfernen der nicht gebundenen Fluoreszenzfarbstoffe vom festen Träger.

Ferner können die Makromoleküle in Schritt a) mit einem Fluoreszenzfarbstoff markiert sein, so dass Schritt c) und c') entfallen können. In diesem Fall kann nach Schritt a) gegebenenfalls der Schritt a') eingefügt werden: Entfernen nicht gebundener Makromoleküle.

Die Makromoleküle können gleich- oder verschiedenartig (identisch oder nicht identisch) sein. Ferner können an die Makromoleküle gleiche oder verschiedenartige Analyten gekoppelt sein. Die vorliegende Erfindung betrifft somit ferner ein Verfahren, in dem entweder gleichartige Makromoleküle verwendet werden, an die gleichartige oder verschiedenartige Analyten gekoppelt sind, oder in dem verschiedenartige Makromoleküle verwendet werden, an die gleichartige oder verschiedenartige Analyten gekoppelt sind. Die Anzahl der verschiedenartigen Analyten ist nicht begrenzt, vielmehr hängt die Anzahl der Analyten von der Größe des Makromoleküls und von den Kopplungsstellen, an den die Analyten angehängt werden, ab.

Die mit dem erfindungsgemäßen Verfahren nachzuweisenden Analyten können Proteine, z.B. Antikörper, Honnone, z.B. Wachstumshormone wie Somatotropin, Polypeptide, insbesondere mit einem Molekulargewicht von kleiner etwa 5000 Dalton, Antibiotika, z.B. β-Lactame, Cephalosporine, Sulfonamide, Tetracyline und andere, Betäubungsmittel, insbesondere illegale Betäubungsmittel, z.B. Tetrahydrocannabinol, Cocain, Morphine, Amphetamin, Metamphetamine und andere, Vitamine, insbesondere Vitamingruppen A - U, z.B. Vitamin B12, Biotin und andere, anabole Steroide wie Testosteron, nichtsteroidale Androgene wie Zeranol und andere, Pesticide, z.B. Atrazin, PCB und andere, Dopingmittel, z.B. Rauschgifte wie Amphetamin, Weckamine wie Ephedrin, Psychopharmaka, z.B. 1,4-Benzodiazepine, und andere, oder potentielle biologische Kampfstoffe, z.B. Anthrax, Botulinumtoxin und andere, sein. Vorzugsweise sind die nachzuweisenden Analyten niedermolekulare Analyten, besonders mit einem Molekulargewicht kleiner etwa 5000 Dalton, insbesondere kleiner etwa 1000 Dalton.

Für das erfindungsgemäße Verfahren werden die nachzuweisenden Analyten an ein Makromolekül gekoppelt. Um die Sensitivität des Assays zu erhöhen, werden mindestens zwei Analyten pro Makromolekül gekoppelt, jedoch ist eine obere Grenze der Anzahl der Analyten pro Makromolekül nicht gegeben. Vielmehr hängt die Anzahl der Analyten von der Größe des Makromoleküls und von den Kopplungsstellen, an den die Analyten angehängt werden, ab. Im Fall der Kopplung an Nukleinsäuren, z.B. Oligonukleotide, kann der Kopplungsgrad daher über die Abfolge der für eine Kopplung geeigneten Basen variiert werden. Werden als Makromoleküle Polypeptide verwendet, beeinflußt ebenfalls die Häufigkeit des Auftretens für die Kopplung geeigneter Aminosäuren in der Prilmärsequenz den Kopplungsgrad. Bei einer Abfolge immer des selben Nukleotids oder der selben Aminosäure, an die gekoppelt werden kann, bestimmt das molare Verhältnis von Makromolekül zu Analyt den Kopplungsgrad. Das molare Verhältnis kann dabei zwischen 1:2 und etwa 1:10 variieren. Molare Verhältnisse von >1:10 können ebenfalls ausreichend sein und ergeben sich je nach Tertiärstruktur bei der Kopplung an Proteine.

Die für das erfindungsgemäße Verfahren geeigneten Makromoleküle können synthetische Kunststoffe, z.B. Melaminharze, Polyaminosäuren, z.B. Polylysin oder Polyglutamat, Peptide, Polypeptide, Proteine, z.B. Antikörper, Peptidnukleinsäuren oder Nukleinsäuren, z.B. DNA oder RNA, sein. Die Nukleinsäuren können einzelsträngig (ss) oder doppelsträngig (ds) verwendet werden. Wird DNA verwendet, ist die Länge und die Sekundärstruktur der DNA von entscheidendem Einfluss auf die Empfindlichkeit des Nachweises. Doppelsträngige und hochmolekulare DNA lässt sich zwar in der Regel leichter anfärben als kurze, einzelne DNA-Stränge, jedoch können die Analyten über die in einzelsträngiger DNA freiliegenden primären Aminogruppen von Adenin, Guanin und Cytosin einfacher gekoppelt werden. Daher ist einzelsträngige DNA als Makromolekül für das erfindungsgemäße Verfahren bevorzugt. Insbesondere bevorzugt sind Oligonukleotide, wobei Oligonukleotide aus Kostengründen mit einer Länge im Bereich von etwa 40 - 80 Basen bevorzugt sind. Oligonukleotide mit einer Länge von weniger als etwa 40 Basen sind ebenfalls geeignet, erzeugen jedoch keine optimale Signalverstärkung. Oligonukleotide mit einer Länge von mehr als etwa 80 Basen sind ebenfalls geeignet, sind jedoch aus Kostengründen nicht bevorzugt. Gegebenenfalls können die mit den Analyten gekoppelten Oligonukleotide mit einem komplementären Oligonukleotid hybridisiert werden, um die Signalstärke einer doppelsträngigen DNA zu erhalten. Um eine Selbsthybridisierung von einzelsträngigen Oligonukleotiden zu vermeiden, wird eine Sequenz aus nichtkomplementären Basen bevorzugt.

Die Kopplung der Analyten an die Makromoleküle erfolgt vorzugsweise über primäre Aminogruppen, insbesondere über die Aminogruppen der Nukleinsäurebasen. Die Kopplung an Peptide oder Proteine kann auch über Sulfhydrylgruppen und Carboxygruppen sowie im Falle glycosilierter Proteine über den Zuckerrest erfolgen. Die Kopplungen erfolgen dabei insbesondere über Glutardialdehyd, Succinimidester, Sulfo-Succinimide, Arylazide, Phenylazide, Maleimide nach vorheriger Umwandlung der Amino- in eine Sulfhydrylguppe, Hydrazide, Pyridilthioverbindungen nach Umwandlung der Amino- in eine Sulfhydrylgruppe, Imidoester, Carbodiimide, vinylsulfon-reaktive Gruppen enthaltende Verbindungen nach Umwandlung der Aminogruppe in eine Sulfhydrylgruppe, Boronsäure enthaltende Verbindungen zur Kopplung an cis-Diole in Ribonucleotiden anstelle der Aminogruppe in den Basen, Umwandlung der primären Aminogruppen in Carboxylgruppen und anschließende Kopplung, Hydroxyphenylazide, Isocyanate, Azidomethylcumarine, Nitrophenylazide, Bromoacetylverbindungen, Perfluoroazidverbindungen, Iodoacetylverbindungen, Psoralene, Iodoacetatverbindungen, biotinhaltige Verbindungen, Hydroxymethylphosphine, sowie Aldehyd und Hydrazid aktivierte Dextrane. Hierbei können Spacer oder Polylinker in unterschiedlicher Länge zwischen den zu koppelnden Molekülen eingebracht werden, die auch die Löslichkeiten der gekoppelten Moleküle in organischen Lösungsmitteln beeinflussen können.

Durch die Wahl der Nukleinsäuresequenz kann der Grad der Kopplung eines Analyten variiert werden. Für Analyten mit einem Molekulargewicht von etwa 300 Dalton hat sich gezeigt, dass eine Kopplungsmöglichkeit an jede fünfte Base im Molekül ausreichend ist. Jedoch können auch mehr oder weniger Kopplungen vorliegen. Die Länge des Oligonukleotids und der Konjugationsgrad nehmen Einfluss auf die Sensitivität des Nachweissystems und kann jeweils empirisch ermittelt werden.

Die vorliegende Erfindung betrifft ferner ein Verfahren, in dem die zu der Probe zugegebenen Makromoleküle mit verschiedenen Analyten gekoppelt sind. Dabei können sowohl gleichartige Makromoleküle mit verschiedenen Analyten, als auch verschiedenartige Makromoleküle mit jeweils verschiedenen Analyten gekoppelt werden. Dieses Verfahren kann z.B. zum Nachweis von mehreren Analyten in einem Mehrfachtest (Array) eingesetzt werden. Vorzugsweise besteht ein Mehrfachtest aus identischen (gleichartigen) Makromolekülen, an die verschiedene Analyten gekoppelt sind. Dabei müssen die Analyten im Verhältnis der jeweilig gewünschten Nachweisempfindlichkeit zur Kopplung mit dem Makromolekül eingesetzt werden. Werden als Makromoleküle Oligonukleotide verwendet, können die Oligonukleotide mit verschiedenen Analyten vorzugsweise hergestellt werden, indem bei der Synthese der Oligonuldeotide Nukleotide verwendet werden, an die vor der Synthese unterschiedliche Analyten gekoppelt wurden.

Die für den Nachweis erforderlichen Fluoreszenzfarbstoffe können entweder in Lösung der mit den Makromolekülen inkubierten Probe zugesetzt werden oder bereits an die Makromoleküle gekoppelt sein. Aus der Molekularbiologie sind Fluoreszenzfarbstoffe bekannt, mit denen DNA im Bereich weniger Pikogramm nach Anregung mit der geeigneten Wellenlänge auch mit dem menschlichen Auge hochempfindlich nachgewiesen werden kann, wenn diese durch entsprechende Maßnahmen (z.B. durch Gelelektrophorese oder Fraktionierung im CsCl-Gradienten) auf einen eng begrenzten Bereich fokussiert wird.

Gängige Farbstoffe zur Färbung von DNA sind das zu den Phenantrinen gehörende Ethidiumbromid, das nach Anregung im UV-Bereich im Roten emittiert oder die zu den Cyaninen gehörenden SYBR-Farbstoffe (SybrGreen bzw. SybrGold) der Fa. Molecular Probes, die sich mit UV und einem Maximum von 485 nm anregen lassen und bei 530 nm emittieren. Beide Farbstoffe sind zur Markierung von einzelsträngiger DNA oder RNA gleichermaßen geeignet, wobei Ethidiumbromid aufgrund seiner Cancerogenität und der für die ungeschützten Augen schädlichen UV-Anregung weniger für ein Vor-Ort-Testverfahren geeignet ist. Zur Markierung von doppelsträngiger DNA oder RNA geeignet sind weiterhin interkalierende Farbstoffe, wie die Phenantrine (z.B. Propidiumjodid, Hexidiumjodid, Dihydroethidium, Ethidiumhomodimere, Ethidiummonazid) und Acridine (z.B. Acridinorange) sowie die in der kleinen DNA-Furche bindenden Indole und Imidazole (z.B. Bisbenzimid-Farbstoffe der Fa. Hoechst) und andere Farbstoffe wie 7-Aminoactinomycin D, Actinomycin D und Hydroxystilbamidin.

Eine bevorzugte Ausführungsform des erfindungsgemäßen Verfahrens besteht darin, dass durch Hybridisierung von Oligonukleotiden mit komplementären DNA-Sequenzen doppelsträngige DNA entsteht, die im Falle der Verwendung interkalierender Farbstoffe mehr Fluoreszenzfarbstoff binden kann, wodurch die Empfindlichkeit des erfindungsgemäßen Verfahrens erhöht wird.

Zur Färbung von Polyaminosäuren eignen sich linkergekoppelte Fluorophore, z.B. n-Hydroxy-Succinimidester aller gängiger Fluoreszenzfarbstoffe, z.B. der Fa. Molecular Probes. Diese Farbstoffe zeigen eine große Variabilität der Fluorophore, z.B. von den blau fuoreszierenden Farbstoffen Cascade Blue, Marina Blue und Alexa Fluor 350 über die grün fluoreszierenden Faarbstoffe Alexa Fluor 488, Alexa Fluor 532, Alexa Fluor 546, Alexa Fluor 568, zu den rot fluoreszierenden Farbstoffen Alexa Fluor 594, Texas Red and Texas Red-X und den dunkelrot fluoresziemden Farbstoffen Alexa Fluor 633, Alexa Fluor 647, Alexa Fluor 660, Alexa Fluor 680 und LaserPro IR 790dyes sowie den Phycobilinen.

Die Aufzählung der Farbstoffe stellt keine abschließende Liste dar sondern wird hier nur beispielhaft genannt. Dem Fachmann ist ersichtlich, dass auch andere Farbstoffe verwendet werden können, die die für das Verfahren verwendeten Makromoleküle anfärben.

Die für das erfindungsgemäße Verfahren verwendeten festen Träger können entweder lichtdurchlässige feste Träger, z.B. Objektträger aus Glas bzw. polymeren Kunststoffen, wie z.B. Polystyrol und andere, oder lichtundurchlässige Träger wie Kunststoffteststreifen die mit Membranen, z.B. Nitrozellulose, Nylon und andere, beschichtet sind, sein. In einer bevorzugten Ausführungsform kann der feste Träger auch eine durchsichtige Säule sein, die mit antikörperbindenden Materialien gefüllt ist, oder ein stabförmiger Kunststoffträger, der über chemische Modifkationen, z.B. Aminierung und andere, oder physikalische Modifkationen, z.B. Einschmelzen von Eupergit und andere, gegeignet ist Antikörper zu binden.

Die auf einem festen Träger gekoppelten Einfangmoleküle können alle Arten von Molekülen sein, die die nachzuweisenden Analyten spezifisch binden können. Bevorzugte Einfangmoleküle sind Antikörper, Rezeptoren, molekular geprägte Polymere (molecular imprints) und deren Konterparts wie Antigene, Liganden, funktionelle Monomere bzw. vernetzte Monomere (printing molecules).

Die Einfangmoleküle können auf speziell vorbeschichtete, kommerziell erhältliche Objektträger, z.B. Epoxy- oder Aldehyd-Slides (Quantifoil Micro Tools GmbH, Jena), Euray (Exiqqon, Vedbaek, Dänemark) und andere gekoppelt werden, wobei nach den Anweisungen der Hersteller vorgegangen werden kann. Ferner eignen sich auch eigenbeschichtete Objektträger, z.B. mit oxiranhaltiger Dispersion (Präparat 2879, Röhm Pharma, Darmstadt). Dazu kann die oxiranhaltige Dispersion auf einem Objektträger ausgestrichen und einige Stunden bei Raumtemperatur getrocknet werden. Um ein mit dem Auge gut sichtbares Signal zu erhalten, werden die spezifischen Antikörper in einem Volumen von etwa 0,01 bis etwa 10 µl auf die vorbereiteten Objektträger aufgespottet. Das Volumen variiert je nach Beschichtung des Objektträgers, der Anzahl der aufzuspottenden Antikörper und der gewünschten Größe des Signals. Vorzugsweise liegen die Volumina zwischen etwa 0,1 und etwa 0,5 µl pro Spot, bei einem Titer größer 1 der Antikörperlösung. Die aufgespotteten Antikörper werden vorzugsweise mehrere Stunden bei 37°C getrocknet, anschließend können die freien, nicht bespotteten Flächen der Objektträgerbeschichtung mit Casein oder anderen geeigneten Proteinen wie BSA (jeweils 1-10 %) in physiologischen Puffern, z.B. PBS oder Tierseren (Schwein, Huhn, etc. 10% in PBS) nach allgemeinen Vorschriften der Immundetektion, z.B. Application Guide Pall Gelman Laboratory: Transfer and Immobilization Procedures for Pall Gelman Laboratory Membranes, geblockt werden. Nicht gebundenes Protein wird abgewaschen, die Objektträger sind nun aktiviert und können für den Nachweis des antikörperspezifischen Analyten eingesetzt werden. In einer spezifischen Ausführungsform sind in einem Testgefäß 10 - 100 ng eines gefriergetrockneten, analytgekoppelten Oligonukleotids vorgelegt. Dieses Oligonukleotid wird mit der Probe, z. B. mit einem Milliliter einer den freien Analyten enthaltenden Probe gelöst und der aktivierte Objektträger in der Testflüssigkeit für einen definierten Zeitraum, je nach Affinität des Antikörpers zwischen 0,5 bis 120 Minuten bei einer Umgebungstemperatur von 5 bis 35°C inkubiert.

Während der Inkubationszeit erfolgt die Kompetition der freien und der an das Makromolekül gebundenen Analyten um die Bindungsstellen der Antikörper, wobei die Empfindlichkeit des Tests für den freien Analyten über die Menge des makromolekülgebundenen Analyten eingestellt werden kann. Je nach Wahl des Fluoreszenzfarbstoffes kann das Makromolekül, z.B. das Oligonukleotid schon vorgefärbt sein oder die Färbung wird für einen definierten Zeitraum nach Inkubation und Spülen des Objektträgers z.B. in einem separaten Testgefäß mit definierter Farbstoffkonzentration notwendig. Zur Reduzierung des Hintergrundes kann überschüssiger Farbstoff nach der Färbung entfernt werden. Dies kann entweder mechanisch durch Abstreifen, z.B. an einer Gummilippe, oder mittels eines definierten Volumens einer Spülflüssigkeit erfolgen. Die Zusammensetzung der Spülflüssigkeit hängt von der Stabilität der gebundenen Einfangmoleküle ab, wobei bevorzugt wässrige Lösungen mit für das verwendete Einfangmolekül und den für die weiteren Reaktionspartnern geeigneten Puffereigenschaften Anwendung finden können. Anschließend erfolgt der Nachweis der makromolekülgebundenen Analyten durch Anregung der Fluoreszenzfarbstoffe von unten durch den Objektträger. In einer bevorzugten Ausführungsform wird das als Makromolekül verwendete Oligonukleotid mit SYBR-Gold (Molecular Probes) gefärbt, mit einem Blaulichttransilluminator (450 nm) angeregt und die Fluoreszenzstrahlung durch einen Orange-Filter (500 - 510 nm) für das menschliche Auge sichtbar gemacht.

Für ein vor Ort einsetzbares Nachweissystem wird bevorzugt ein batteriebetriebener Blaulichttransilluminator verwendet, der über einen angehängten Orange-Filter und eine darüber gesetzte Verdunkelung verfügt. In den Abmessungen entspricht die Schirmfläche des Transilluminators der Fläche eines für den Test verwendeten Objektträgers. Der Filter ist so über die Schirmfläche montiert, dass der Objektträger zwischen Schirmfläche und Filter geschoben und durch Federklemmen oder vergleichbare Halterungsmechanismen fixiert werden kann. Um die Fluoreszenzstrahlung in voller Stärke mit dem Auge wahrnehmen zu können, ist der Filter auf der abstrahlenden Seite mit einer an die Gesichtsform angepassten lichtundurchlässigen Maske verbunden, die vor die Augenpartie des Auswertenden passt und Tageslicht oder störendes Licht aus der Umgebung abhält.

Das Verfahren kann in der Lebens- und Futtermittelkontrolle, z.B. zum Nachweis von Schadstoffen, z.B. Antibiotika, Pestizide, Hormone und andere verwendet werden. Hier existieren größtenteils bislang nur Labormethoden, die so zeitaufwendig sind, dass die Lebens- oder Futtermittel schon konsumiert sind, bis das Untersuchungsergebnis feststeht. Die für immunologische Schnelltests bislang verwendeten Markierungen eignen sich aber nur bedingt für die Auswertung vor Ort, wie bei Kunstlicht im Supermarkt oder bei Kontrollen in schlecht beleuchteten Ställen. Dieses Problem wird mit der vorliegenden Erfindung ausgeräumt.

Weitere Anwendungsbeispiele, für die das erfindungsgemäße Verfahren erhebliche Vorteile bringt, liegen im diagnostischen Bereich. Sowohl in der veterinärmedizinischen Diagnostik bietet das Verfahren unter Stallbedingungen Vorteile als auch in der humanmedizinischen Notfalldiagnostik, bei der Schnelltests rund um die Uhr im Rettungswagen durchgeführt werden müssen.

Darüber hinaus ist das erfindungsgemäße Verfahren sehr gut geeignet für Verkehrskontrollen zur Ermittlung von drogenbeeinflußten Autofahrern. Das Problem des Drogenkonsums ist oft mit nächtlichen Besuchen von Diskotheken verbunden. Daher finden entsprechende Kontrollen überwiegend unter ungünstigen Lichtbedingungen statt. Die Auswertung von Schnelltests gegen illegale Drogen und Barbiturate kann wesentlich erleichtert werden.

Die folgenden Beispiele erläutern die Erfindung und sind nicht als einschränkend aufzufassen.

### (1) Herstellung eines Oligonukleotids

Um eine gezielte Kopplung zu erreichen, wurde eine Sequenz aus Thymidin und Guanosin synthetisiert, da an Thymin aufgrund einer fehlenden primären Aminogruppe kein Analyt gekoppelt werden kann. Die Kopplung erfolgte also über die primäre Aminogruppe des Guanins, wobei Cytosin zur Kopplung genauso geeignet wäre. Das Oligonukleotid mit der Sequenz (TTTTG)₁₆ wurde mit salzfreiem, standardmäßigem Reinheitsgrad bei Qiagen Operon in Auftrag gegeben und in lyophilisierter Form ausgeliefert. Aus dem Lyophilisat wurde eine Stocklösung von 10 mg/ml in PBS (Phosphate Buffered Saline, 137 mM NaCl, 2,7 mM KCl, 8mM Na₂HPO₄·2H₂O; 1,5 mM KH₂PO₄) angesetzt und in Aliquots bei -20°C gelagert.

### (2) Kopplung von Analyten

### (a) Ampcillin an ein Oligonukleotid,

Zur Aktivierung wurde 1,5 mg Ampicillin (5mg/ml in 100 mM c) unter ständigem Rühren tropfenweise mit 25 mg/ml m-Maleimidobenzoyl-N-hydroxysulfosuccinimidester (in H₂O) bis zu einer Enkonzentration von 5 mg/ml versetzt und anschließend 30 min. bei Raumtemperatur gerührt. Der Überschuß an Maleimidgruppen wurde durch eine anschließende einstündige Inkubation nach Zugabe von 1 M Dithiothreitol bis zu einer Endkonzentration von 35 mM inaktiviert. Zur Kopplung wurden dann 3,9 mg des Oligonukleotids (2 mg/ml in PBS) in den Ansatz gegeben, gemischt und 3 Stunden bei Raumtemperatur inkubiert. In einer anschließenden Dialyse gegen PBS, über Nacht mit vierfachem Pufferwechsel bei 4°C, wurde nicht gekoppeltes Ampicillin und m-Maleimidobenzoyl-N-hydroxysulfosuccinimidester aus dem Reaktionsgemisch entfernt.

### (b) Ampicillin und AlexaFluor 488 an Polylysin

Wiederum 2 mg Ampicillin (5 mg/ml in 100 mM Natriumhydrogenphosphat in H₂O, pH 7,2) wurden zur Aktivierung unter Rühren tropfenweise mit 2 mg m-Maleimidobenzoyl-N-hydroxysulfosuccinimidester (25 mg/ml in H₂O) versetzt und 30 Min. bei Raumtemperatur gerührt. Der Überschuss an Maleimidgruppen wurde durch eine einstündige Inkubation mit 1 M Dithiothreitol (Endkonzentration 35 mM) inaktiviert. Gleichzeitig wurde 1 mg Polylysin (3-15 kDa) in 0,1 M Natriumhydrogencarbonat gelöst (2mg/ml); 4,4 µl AlexaFluor 488 wurden mit bidestilliertem Wasser auf 100 µl verdünnt, in Aliquots von 10 µl - 20 µl unter Rühren zugegeben und 1 Stunde im Dunkeln bei RT gerührt. Freier Farbstoff wurde mit einem 3K Zentrifugenkonzentrator (Pall) abgetrennt, indem 20 Minuten bei RT zentrifugiert und einmal mit 500 µl PBS gewaschen wurde. Das Konzentrat wurde in 500 µl PBS aufgenommen. Anschließend wurde aktiviertes Ampicillin zugegeben und 3 Stunden im Dunkeln bei Raumtemperatur gerührt. Durch eine Dialyse gegen PBS bei 4°C über Nacht und mit vierfachem Pufferwechsel wurde nicht gekoppeltes Ampicillin und m-Maleimidobenzoyl-N-hydroxysulfosuccinimidester entfernt.

### (3) Beschichtung der Objektträger

Objektträger, z.B. "SuperFrost" der Fa. Menzel, wurden mit 5 µl einer oxiranhaltigen Dispersion (Präparat 2879, Röhm Pharma, Darmstadt) durch gleichmäßiges Ausstreichen mit einem Glas- oder Plastikstäbchen beschichtet und anschließend bei Raumtemperatur für mindestens 2 Stunden getrocknet. Das Auftragen der Antikörper erfolgte in Verdünnungen (10⁰, 10⁻¹, 10⁻², 10⁻³) manuell mit einer Pipette, wobei Volumina von 0,1 bis 1,0µl so aufgetragen wurden, daß die Pipettenspitze den Objektträger nicht berührte. Als Verdünnungs- bzw. Auftragslösung hat sich PBS als sehr geeignet erwiesen. Die Fixierung der Antikörper auf dem Objektträger erfolgte bei 37°C über 2 - 3 Stunden oder bei Raumtemperatur über Nacht. Anschließend wurden die beschichteten Objektträger bei Raumtemperatur 30 Minuten in Milchpuffer (10mM Tris-HCl, pH 7,4, 150mM NaCl, 0,3% Casein nach Hammarsten, 0,05% Tween 20, 0,005% NaN₃ (fakultativ)) oder 100mM Tris-HCl, pH 9 mit 50mM Ethanolamin und 0,1% SDS oder Schweineserum (1:10 mit PBS verdünnt) blockiert.

### (4) Testdurchführung

### (a) mit ampicillingekoppelten Oligonukleotiden

Die Objektträger wurden für 20 min. bei Raumtemperatur in einer flachen Schale liegend mit 20 ml 0,5% Rinderserumalbumin, 0,05% Tween 20 in PBS mit verschiedenen Konzentrationen Ampicillin und des ampicillingekoppelten Oligonukleotids inkubiert und danach 3x kurz mit 11,5 mM NaCl, 0,23 mM KCl, 0,66 mM Na₂HPO₄ x 2H₂O, 0,13 mM KH₂PO₄, 0,04% Tween 20 in H₂O gespült. Anschließend wurden die Objektträger 1 Minute mit SYBRGold (Molecular Probes) in einer Verdünnung von 1:30.000 in TAE-Puffer (40mM Tris-HCl, pH 7,5, 20 mM Natrium-Acetat, 1mM EDTA) gefärbt. Unmittelbar nach der Färbung wurden die Objektträger noch einmal wie vorher gewaschen, sofort auf einen Blaulichttransilluminator gelegt und ausgewertet. Ein negatives Ergebnis, d.h. die Abwesenheit des Analyten, wird durch ein Fluoreszenzsignal angezeigt. Mit zunehmender Konzentration des Analyten nimmt die Signalintensität ab. Die vollständige Verdrängung des Ampcillinkonjugates erfolgte bei einer Konzentration von 100 ng/ml Ampicillin.

### (b) mit Ampicillin und Fluorphor gekoppeltem Polylysin

Beschichtete Objektträger wurden wie unter (a) beschrieben mit verschiedenen Konzentrationen Ampicillin und dem Ampicillin-AlexaFluor488-gekoppelten Polylysin für 20 Minuten lichtgeschützt inkubiert und wie unter (a) gespült. Die Objektträger konnten dann ohne weitere Behandlung auf einen Blaulichttransilluminator gelegt und ausgewertet werden. Die Abwesenheit des Analyten wurde durch ein Fluoreszenzsignal angezeigt. Zunehmende Konzentrationen des freien Ampcillins verdrängten das Ampicillin-Konjugat bis bei 1 mg/ml Ampicillin kein Signal mehr zu sehen war.

### (5) Ergebnisse vergleichender Versuche

| Nachweis von (freier Analyt) | Einfang-molekül | Makromolekül (gekoppelter Analyt) | Fluoreszenzfarbstoff | Testmatrix | bislang erreichte Test-empfindlichkeit |
|---|---|---|---|---|---|
| Ampicillin | Anti-Ampicillin-Antikörper | ssDNA-Ampicillin | SybrGold | Puffer | < 1 µg/ml |
| Ampicillin | Anti-Ampicillin-Antikörper | ssDNA-Ampicillin | SybrGold | Milch | < 10µg/ml |
| Ampicillin | Anti-Ampicillin-Antikörper | ssDNA-Ampicillin | Ethidium-bromid | Puffer | < 1 µg/ml |
| Ampicillin | Anti-Ampicillin-Antikörper | dsDNA-Ampicillin | Ethidum-bromid | Puffer | < 0,1 µg/ml |
| Ampicillin | Anti-Ampicillin-Antikörper | Polylysin-Ampicillin | AlexaFluor-488 | Puffer | < 1 µg/ml |
| Biotin | Anti-Biotin-Antikörper | ssDNA-Biotin | SybrGold | Milch | < 1 µg/ml |
| THC | Anti-THC-Antikörper | Polylysin-THC | AlexaFluor-488 | Puffer | < 1 µg/ml |
| Hautpilz-epitope | Pilzantigen + Anti-Pilzantikörper | Anti-Ig-Antikörper | AlexaFluor-488 | Kaninchenserum | nicht bestimmt |

## Patentansprüche

1. Verfahren zum Nachweis von Analyten, umfassend die Schritte:
a) Inkubieren einer Probe mit Makromolekülen, an die jeweils mindestens 2 Moleküle des in der Probe nachzuweisenden Analyten gekoppelt sind,
b) anschließendes Inkubieren der Probe mit einem festen Träger, an den Einfangmoleküle gegen den nachzuweisenden Analyten gekoppelt sind,
c) Zugabe eines die Makromoleküle anfärbenden Fluoreszenzfarbstoffes und
d) Nachweis von in der Probe vorhandenen Analyten durch Anregung des Fluoreszenzfarbstoffes.

2. Verfahren nach Anspruch 1, nach Schritt c) ferner umfassend Schritt c'): Entfernen der nicht gebundenen Fluoreszenzfarbstoffe vom festen Träger.

3. Verfahren zum Nachweis von Analyten, umfassend die Schritte:
a) Inkubieren einer Probe mit Fluoreszenzfarbstoff markierten Makromolekülen, an die jeweils mindestens 2 Moleküle des in der Probe nachzuweisenden Analyten gekoppelt sind,
b) anschließendes Inkubieren der Probe mit einem festen Träger, an den Einfangmoleküle gegen den nachzuweisenden Analyten gekoppelt sind,
c) Nachweis von in der Probe vorhandenen Analyten durch Anregung des Fluoreszenzfarbstoffes.

4. Verfahren nach Anspruch 3, nach Schritt a) ferner umfassend Schritt a'): Entfernen der nicht gebundenen Makromoleküle.

5. Verfahren nach einem der vorhergehenden Ansprüche, wobei die Makromoleküle Nukleinsäuren, Peptidnukleinsäuren, Polyaminosäuren sind

6. Verfahren nach einem der vorhergehenden Ansprüche, wobei die Makromoleküle einzelsträngige Oligonukleotide mit einer Länge im Bereich von 40 bis 80 Nukleotiden sind.

7. Verfahren nach einem der vorhergehenden Ansprüche, wobei die Makromoleküle gleichartig oder verschiedenartig sind.

8. Verfahren nach einem der vorhergehenden Ansprüche, wobei die Analyten ein Molekulargewicht von weniger als 5000 Dalton aufweisen.

9. Verfahren nach einem der vorhergehenden Ansprüche, wobei der Fluoreszenzfarbstoff aus der Gruppe der Phenantrine, Acridine, der SYBR-Farbstoffe oder der Fluorophore ausgewählt ist.

10. Verfahren nach einem der vorhergehenden Ansprüche, wobei der feste Träger lichtdurchlässig ist und das Nachweisverfahren mittels eines Durchlichtverfahrens durchgeführt wird.

## Claims

1. Method for detecting analytes comprising the following stages:
a) Incubation of a sample with macromolecules, to each of which at least 2 molecules of the analyte to be detected in the sample are coupled;
b) Subsequent incubation of the sample with a solid carrier, to which capture molecules for the analyte to be detected are coupled;
c) Addition of a fluorescence dye to stain the macromolecules;
d) Detection of the analytes present in the sample by excitation of the fluorescence dye.

2. Method according to claim 1 comprising, after stage c), a further stage c'): Removal of the non-bound fluorescence dye from the solid carrier.

3. Method for detecting analytes comprising the stages:
a) Incubation of a sample with fluorescence-dye-marked macromolecules, to each of which at least 2 molecules of the analyte to be detected in the sample are coupled;
b) Subsequent incubation of the sample with a solid carrier, to which capture molecules for the analyte to be detected are coupled;
c) Detection of analytes present in the sample by excitation of the fluorescence dye.

4. Method according to claim 3 comprising, after stage a), a further stage a'): Removal of the non-bound macromolecules.

5. Method according to any one of the preceding claims, wherein the macromolecules are nucleic acids, peptide nucleic acids, polyamino acids.

6. Method according to any one of the preceding claims, wherein the macromolecules are single-strand oligonucleotides of a length within the range from 40 to 80 nucleotides.

7. Method according to any one of the preceding claims, wherein the macromolecules are identical or non-identical.

8. Method according to any one of the preceding claims, wherein the analytes have a molecular weight of less than 5000 Dalton.

9. Method according to any one of the preceding claims, wherein the fluorescence dye is selected from the group of phenanthrenes, acridines, SYBR dyes or fluorophores.

10. Method according to any one of the preceding claims, wherein the solid carrier is permeable to light and the detection method is implemented by means of a transmitted-light method.

## Revendications

1. Procédé de détection d'analytes, comprenant les étapes consistant à :
a) incuber un échantillon avec des macromolécules sur lesquelles sont couplées respectivement au moins 2 molécules d'analytes à détecter dans l'échantillon,
b) incuber ensuite l'échantillon avec un support solide sur lequel sont couplées des molécules de capture des analytes à détecter,
c) ajouter un colorant fluorescent colorant les macromolécules et
d) détecter les analytes présents dans l'échantillon par excitation du colorant fluorescent.

2. Procédé selon la revendication 1, comprenant en outre après l'étape c), l'étape c') consistant à éliminer le colorant fluorescent non lié au support solide.

3. Procédé de détection d'analytes, comprenant les étapes consistant à :
a) incuber un échantillon avec des macromolécules marquées par un colorant fluorescent, sur lesquelles sont couplées respectivement au moins 2 molécules d'analytes à détecter dans l'échantillon,
b) incuber ensuite l'échantillon avec un support solide sur lequel sont couplées des molécules de capture des analytes à détecter,
c) détecter les analytes présents dans l'échantillon par excitation du colorant fluorescent.

4. Procédé selon la revendication 3, comprenant en outre après l'étape a) l'étape a') consistant à éliminer les macromolécules non liées.

5. Procédé selon l'une quelconque des revendications précédentes, dans lequel les macromolécules sont des acides nucléiques, des acides peptides nucléiques, des peptides.

6. Procédé selon l'une quelconque des revendications précédentes, dans lequel les macromolécules sont des oligonucléotides à simple brin, présentant une longueur de l'ordre de 40 à 80 nucléotides.

7. Procédé selon l'une quelconque des revendications précédentes, dans lequel les macromolécules sont de même nature ou de nature différente.

8. Procédé selon l'une quelconque des revendications précédentes, dans lequel les analytes présentent un poids moléculaire inférieur à 5000 dalton.

9. Procédé selon l'une quelconque des revendications précédentes, dans lequel le colorant fluorescent est choisi dans le groupe des phénantrines, des acridines, des colorants SYBR ou des fluorophores.

10. Procédé selon l'une quelconque des revendications précédentes, dans lequel le support solide est transparent à la lumière et le procédé de détection est réalisé au moyen d'un procédé par transparence.
